# FASCICULE DE BREVET EUROPEEN

(11) **EP 3 897 488 B1**
(45) Date de publication et mention de la délivrance du brevet: **18.01.2023**
(21) Numéro de dépôt: 19829281.5
(22) Date de dépôt: 15.11.2019
(51) Int. Cl.: A61F 9/08, A61H 3/06, G06F 3/01, A61B 5/11, A61B 3/113

(54) **DISPOSITIF MÉDICAL D'AIDE À LA PERCEPTION D'ENVIRONNEMENT POUR DES UTILISATEURS AVEUGLES OU MALVOYANTS**
MEDIZINPRODUKT ZUR VERBESSERUNG DER UMGEBUNGSWAHRNEHMUNG FÜR BLINDE ODER SEHBEHINDERTE BENUTZER
MEDICAL DEVICE FOR IMPROVING ENVIRONMENTAL PERCEPTION FOR BLIND OR VISUALLY IMPAIRED USERS

(30) Priorité: 17.12.2018 FR 1873115
(43) Date de publication de la demande: 27.10.2021
(73) Titulaire: Briand, Pierre, 78140 Vélizy-Villacoublay (FR)
(72) Inventeur: Briand, Pierre, 78140 Vélizy-Villacoublay (FR)
(74) Mandataire: Plasseraud IP
(86) Numéro de dépôt international: PCT/FR2019/052721
(87) Numéro de publication internationale: WO 2020/128173

(56) Documents cités:
- WO-A1-2013/018090
- US-A1- 2007 016 425
- US-A1- 2007 041 600
- US-A1- 2018 249 151

## Description

### Domaine technique

L'invention concerne une aide à la perception d'environnement, en particulier pour des utilisateurs aveugles ou malvoyants.

On entend par « utilisateurs aveugles » des personnes atteintes de cécité durable ou simplement de circonstance, comme par exemple en vue d'une utilisation haptique (dans le noir absolu pour un usage militaire ou autre), ou en complément de réalité virtuelle ou augmentée ou autre.

### Technique antérieure

On connaît de l'état de l'art les implants rétiniens. Il s'agit de dispositifs implantés chirurgicalement au fond de l'un des yeux de la personne aveugle, stimulant électriquement les neurones à destination du cerveau, en reproduisant une image acheminée par radio depuis un capteur vidéo installé sur des lunettes. Mais leurs coûts et la complexité de leur mise en œuvre en limitent la diffusion.

Parallèlement à ces dispositifs, il est également connu de l'état de l'art des dispositifs portables pour aveugles, capables de convertir une image vidéo acquise d'un environnement face au sujet, en une « image tactile » reproduite sur la peau ou des muqueuses du sujet. La version exploitée actuellement comporte une caméra vidéo frontale reliée à une unité d'électrostimulation (de basse intensité), en l'espèce sur la langue de l'utilisateur. Cette restitution pourtant sommaire est cependant exploitable par les aveugles.

Le document US2007016425 divulgue un dispositif selon le préambule de la revendication 1.

Un inconvénient majeur de ces dispositifs est qu'ils perdent nettement en performance lorsque l'objet visé par la caméra est mobile. Typiquement, l'utilisateur doit tourner son buste, son cou, sa tête, vers une direction de provenance d'un son ou autre. A terme, une telle réalisation fatigue finalement l'utilisateur, qui finit par se lasser du dispositif.

### Objet de l'invention

La présente invention vient améliorer la situation.

Elle propose à cet effet un dispositif offrant une aide à la perception spatiale d'un environnement, en mesurant les mouvements des yeux du sujet aveugle et en asservissant en fonction de cette mesure l'image qui est restituée sur la peau.

Ainsi, la présente invention vise un dispositif d'aide à la perception d'environnement pour des utilisateurs aveugles ou malvoyants, le dispositif comportant :
- au moins un jeu d'actuateurs mécaniques destiné à être en contact avec la peau d'un utilisateur,
- au moins une caméra numérique agencée pour acquérir une image numérique courante d'un environnement face à l'utilisateur,
- un circuit de traitement connecté à la caméra pour recevoir des signaux de pixels de l'image numérique acquise et convertir une partie au moins des signaux de pixels en signaux de commande alimentant chacun un actuateur mécanique du jeu d'actuateurs.

En particulier, le dispositif comporte en outre au moins un module de suivi oculaire d'au moins un œil de l'utilisateur pour identifier une direction de regard de l'utilisateur. En outre, le circuit de traitement est agencé pour sélectionner dans l'environnement filmé par la caméra une zone d'image courante acquise qui est fonction de la direction de regard, et pour convertir les signaux de pixels de la zone précitée en signaux de commande alimentant chacun un actuateur du jeu pour stimuler la peau de l'utilisateur.

On entend par « actuateurs mécaniques » typiquement des éléments qui peuvent être arrangés en matrice par exemple, et apte chacun à se déplacer sur réception d'une impulsion électrique. Il peut s'agir ainsi d'éléments piézoélectriques aptes à avoir par exemple :
- un déplacement d'une amplitude fonction du signal électrique qu'ils reçoivent, et/ou encore
- une fréquence de déplacement fonction du signal électrique qu'ils reçoivent.

On peut prévoir alternativement ou combinés à des éléments piézoélectrique des systèmes de type micro-électro-mécanique (ou « MEMS »).

En variante encore, on peut utiliser une émission d'ultrasons focalisés répartis en une matrice plane pour stimuler la peau de l'utilisateur. En variante encore, il peut s'agir d'électrode émettant des impulsions électriques ressenties comme des mini-chocs électriques (dont l'intensité peut caractériser un niveau de gris d'image, et avec une fréquence de répétition caractérisant une couleur particulière, comme on le verra plus loin à titre d'exemple de réalisation).

On entend en outre par « peau » de l'utilisateur ci-dessus aussi bien la peau de l'utilisateur (sur le torse ou autre) que des muqueuses comme le palais, la langue, ou autre, ce qui correspond aussi au terme générique médical de « tégument ». Ainsi, les actuateurs peuvent se présenter comme des électrodes adressant des micro-courants, et logées par exemple dans un faux-palais intrabuccal (comme la partie collée au palais d'un dentier), ce qui n'empêche à l'utilisateur ni de parler ni de manger ni de boire.

On entend en outre par « caméra numérique » le fait que l'image acquise de l'environnement comporte des pixels arrangés typiquement en matrice, et à chaque pixel peut être assigné un signal électrique pour alimenter un actuateur. Par exemple, pour une image monochrome (« Noir et Blanc »), l'intensité du signal électrique alimentant un actuateur peut être proportionnelle à un niveau de gris (de gris foncé-noir à gris clair-blanc) de pixel. En fonction de l'intensité de ce signal électrique, l'actuateur mécanique peut se déplacer avec une amplitude et/ou une fréquence plus ou moins grande.

Ainsi, le dispositif au sens de l'invention permet de restituer une attention de l'utilisateur, focalisée sur une zone particulière de l'environnement, et ce grâce à la mesure de suivi oculaire de l'utilisateur et donc de son regard. Par exemple, si l'utilisateur a entendu un son dans une direction particulière, il peut diriger son regard vers cette direction ce dont le dispositif rend compte en restituant alors une « image tactile » de la zone de l'environnement, sélectionnée dans cette direction.

Pour ce faire, l'invention a dû vaincre un préjugé selon lequel les aveugles perdraient leur faculté de suivi oculaire. Or, pour les aveugles récents au moins (et pour autant qu'ils aient conservé leurs globes oculaires), il a été observé de façon contre-intuitive que les mouvements oculaires des aveugles étaient toujours présents (malgré leur cécité).

Ainsi, la présente invention vise un dispositif se présentant comme un équipement (ou un appareil) animé par un logiciel et destiné à être utilisé à des fins, notamment, d'atténuation d'un handicap (en l'espèce la cécité d'un utilisateur aveugle). A ce titre, le dispositif de l'invention peut être qualifié de dispositif médical.

Dans une réalisation, le circuit de traitement peut être agencé pour sélectionner dans une image courante acquise une zone limitée de l'image courante qui est fonction de la direction de regard, et convertir les signaux de pixels de ladite zone limitée en signaux de commande alimentant chacun un actuateur du jeu pour stimuler la peau de l'utilisateur.

Dans une variante, il peut être prévu que la caméra (pas nécessairement grand-angle) puisse être mobile en rotation pour suivre la direction du regard de l'utilisateur. Néanmoins, il peut être avantageux de mettre en œuvre une caméra fixe (grand-angle) et de sélectionner informatiquement la zone pertinente dans l'image ainsi acquise en grand-angle en fonction de la direction du regard.

On peut prévoir toutefois aussi des caméras grand-angle, et aussi mobile en rotation (au moins azimutale), pour suivre un mouvement des yeux convergeant vers le nez de l'utilisateur (ce qui correspond à une recherche de « vision de près »). En outre, ces caméras peuvent s'auto-focaliser (avec un module de détection de flou), ce qui permet d'offrir à l'utilisateur une perception spatiale de l'environnement, aussi bien dans un espace proche (« vision de près ») que lointain (« vision lointaine »). En-dehors des situations de focalisation et en particulier pour une « vision lointaine », les caméras peuvent être réglées par défaut avec une orientation relative de l'une par rapport à l'autre en respectant au mieux l'orientation physiologique des yeux homolatéraux de l'utilisateur (environ 15° latéralement par rapport au plan sagital, soit donc d'environ 30° pour l'orientation d'une caméra par rapport à l'autre).

Dans une forme de réalisation possible, le dispositif comporte en outre :
- deux jeux d'actuateurs mécaniques destinés à être en contact avec la peau d'un utilisateur, et
- deux modules de suivi oculaire des yeux respectifs de l'utilisateur, pour définir deux zones dans l'image courante et convertir les signaux de pixels de chacune des deux zones en deux jeux respectifs de signaux de commande alimentant les deux jeux respectifs d'actuateurs.

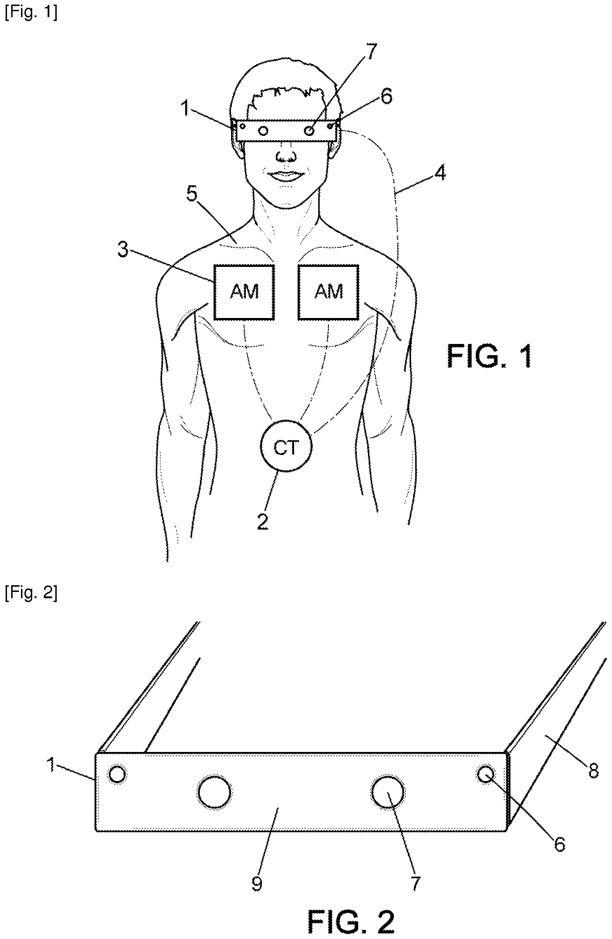

En appliquant un suivi oculaire (ou « eye-tracking » en anglais) à chacun des yeux de l'utilisateur, le dispositif peut apporter, grâce en outre à la paire de jeux d'actuateurs mécaniques, une perception en trois dimensions de l'espace devant le regard de l'utilisateur. En effet, grâce à cette binocularité, tant pour le suivi oculaire (ou « eye-tracking » en anglais) que pour les jeux d'actuateurs mécaniques, le dispositif peut apporter à l'utilisateur une perception en trois dimensions de l'espace regardé. Comme on le verra plus loin en référence à la figure 1, les jeux d'actuateurs sont distincts et séparés spatialement, mais néanmoins le cerveau humain est configuré naturellement pour reproduire une image unique, tridimensionnelle, à partir de deux images distinctes bidimensionnelles (comme avec habituellement les deux yeux de l'utilisateur).

Ainsi, pour une personne aveugle mais supposée ici posséder encore la faculté de mouvements oculaires pour ses deux yeux, l'utilisateur peut recevoir sur sa peau les deux « images tactiles », lesquelles peuvent se confirmer mutuellement pour une perception 3D.

Néanmoins, il s'agit d'une forme de réalisation sophistiquée. On peut prévoir un seul module de suivi oculaire (pour une raison économique ou encore si l'utilisateur n'a plus qu'un seul œil dont on peut mesurer les déplacements de pupille), avec éventuellement deux caméras pour filmer l'environnement face à l'utilisateur (avec éventuellement un angle d'environ 30° entre chaque axe de caméra pour respecter l'orientation physiologique habituelle des axes optiques des yeux). La seule indéterminée possible en utilisant un seul suivi oculaire est la discrimination :
- d'une situation où l'utilisateur regarde une image proche, avec une convergence habituelle des yeux vers le nez, et
- d'une situation où l'utilisateur regarde au loin mais de côté, et vers le nez.

Dans ce cas, les caméras peuvent être « autofocus » et ainsi interpréter distinctement chacune de ces deux situations.

Dans un mode de réalisation de l'invention, la caméra et le module de suivi oculaire, au moins, sont montés sur un support mécanique commun, le module de suivi oculaire étant orienté vers un œil de l'utilisateur (typiquement l'œil homolatéral de l'utilisateur), la caméra étant orientée pour acquérir une image numérique courante d'un environnement face à l'utilisateur. Ainsi, la camera peut être orientée selon l'axe physiologique de l'œil homolatéral de l'utilisateur (ou chaque caméra orientée selon l'axe d'un œil.

Ainsi, la distance entre la caméra et le module de suivi oculaire reste constante dans le temps sur le support, permettant de corréler à tout moment la position du regard de l'utilisateur avec ce que filme la caméra.

Avantageusement, la caméra est de type grand-angle avec une focale entre 10 et 35 mm.

Dans un mode de réalisation de l'invention, le dispositif comprend une liaison sans fil pour la transmission des signaux de commande des actuateurs pour au moins une connexion entre le circuit de traitement et les actuateurs.

La liaison sans fil a pour avantage de connecter par exemple les actuateurs (qui peuvent stimuler le torse ou le dos de l'individu comme décrit plus loin en référence à la figure 1) au reste du dispositif, sans encombrement, et par exemple de mettre en liaison un support mécanique regroupant les modules de suivi oculaire, les caméras et le circuit de traitement, etc., avec les actuateurs mécaniques.

La peau des mains d'un utilisateur étant particulièrement sensible, il peut être prévu, dans une variante, que les actuateurs mécaniques soient intégrés à des gants, les jeux d'actuateurs mécaniques étant placés avantageusement sous forme de patchs respectifs sur la paume des mains de l'utilisateur, par exemple. On prévoit par exemple un gant avec un patch d'actuateurs mécaniques pour chaque main, correspond chacun à une image de l'environnement à percevoir (avec ainsi une reconstruction possible tridimensionnelle). Les caméras, modules de suivi oculaire et circuit de traitement peuvent être montés sur un support commun et reliés sans fils aux patchs des gants.

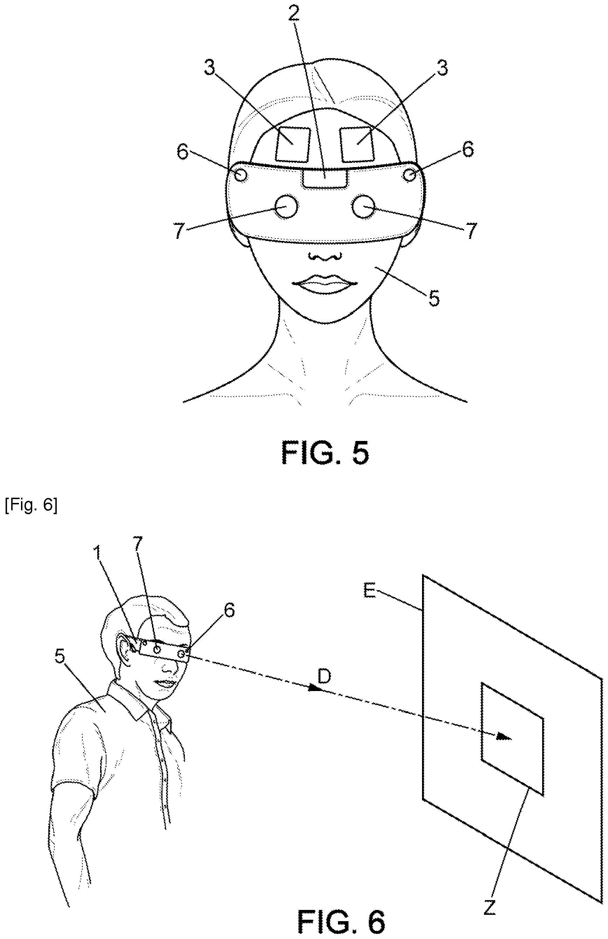

Dans une variante où les actuateurs mécaniques peuvent stimuler une partie de la tête de l'utilisateur (par exemple le front comme illustré sur la figure 5), l'ensemble (caméra, module de suivi oculaire, etc., ainsi que les actuateurs) peut être monté sur un support commun, par exemple sous la forme d'un casque prenant le front et descendant sur les yeux de l'utilisateur.

Dans un mode de réalisation, l'actuateur est agencé pour stimuler la peau de l'utilisateur selon une intensité fonction du signal de commande reçu, et les signaux de commande sont fonctions chacun d'une intensité de couleur de pixel.

On entend par « intensité de couleur de pixel » une intensité lumineuse du pixel pour une couleur donnée.

Par exemple pour une image « noir et blanc », chaque actuateur peut être activé en fonction du niveau de gris du pixel allant de blanc à noir. En variante, on peut prévoir un ensemble de trois actuateurs pour chaque pixel d'une image en couleur, chaque actuateur ayant une intensité ou une fréquence vibratoire fonction du niveau de couleur « rouge, bleu, vert » de ce pixel.

Par exemple, on peut prévoir pour la « restitution » d'une image couleur que l'intensité lumineuse d'un pixel (de foncé à clair par exemple) soit convertie en intensité de mouvement d'actuateur (appuyant ainsi plus fort sur la peau de l'utilisateur), tandis que la fréquence de vibration de chaque actuateur peut être fonction du type de couleur (rouge ou bleu ou vert) assignée à cet actuateur. Par exemple, les actuateurs reproduisant les niveaux de bleu vibrent plus vite que les actuateurs reproduisant le niveau de rouge (le vert ou le jaune étant représentés par des actuateurs ayant une fréquence de vibration intermédiaire).

Ainsi, dans une forme de réalisation où chaque actuateur est mobile en vibration pour stimuler la peau de l'utilisateur par des vibrations cycliques, la fréquence des vibrations d'un actuateur peut être fonction d'un type de couleur de pixel.

Dans une réalisation, la zone d'image peut être de forme générale correspondant à une forme de champ visuel de l'œil suivi par le module de suivi oculaire, et les actuateurs du jeu d'actuateurs peuvent être alors disposés de façon à être répartis selon une forme bidimensionnelle correspondant à cette forme de champ visuel.

Ainsi, on comprendra qu'il est prévu dans le dispositif une correspondance « un à un » d'un pixel de la zone avec un actuateur du jeu. On peut ainsi prévoir un nombre d'actuateurs du jeu correspondant au nombre de pixels dans la zone. On peut prévoir aussi spatialement une correspondance de positions des pixels dans la zone et des actuateurs respectifs dans le jeu d'actuateurs. Néanmoins, il s'agit d'un exemple de réalisation. Par exemple, un actuateur peut être associé à un groupe de quatre pixels de l'image ou plus (avec un niveau de gris moyen par exemple). De même, les actuateurs peuvent ne pas être régulièrement espacés dans le jeu d'actuateurs (car le jeu d'actuateurs peut s'étendre sur des zones corporelles plus ou moins sensibles, les zones plus sensibles pouvant accueillir plus d'actuateurs, typiquement).

Le dispositif peut comporter typiquement un circuit de traitement notamment pour piloter les actuateurs à partir des pixels de la zone d'image acquise. Ce circuit de traitement met en œuvre alors un procédé, que vise en outre la présente invention et comportant les étapes:
- recevoir des signaux de pixels de l'image numérique acquise,
- recevoir une mesure par suivi oculaire d'une direction de regard de l'utilisateur,
- déterminer une délimitation dans l'image acquise d'une zone correspondant à la direction du regard, et sélectionner les signaux de pixels de ladite zone,
- convertir les signaux de pixels de ladite zone en des signaux de commande destinés à alimenter chacun un actuateur du jeu d'actuateurs mécaniques, le nombre de pixels dans ladite zone correspondant à un nombre d'actuateurs que comporte le jeu d'actuateurs mécaniques,
- transmettre les signaux de commande respectifs aux actuateurs dudit jeu.

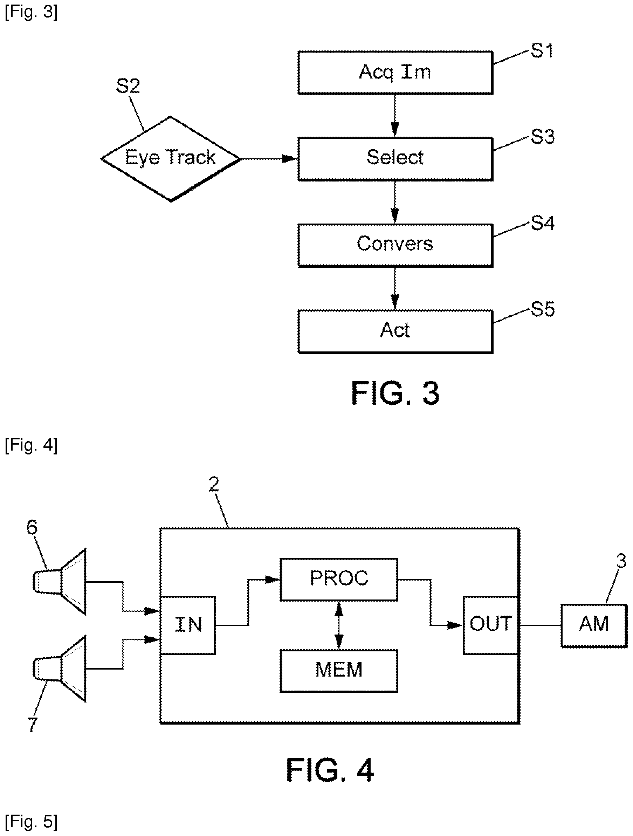

Le circuit de traitement du dispositif peut comporter typiquement (comme illustré sur la figure 4) un processeur et une mémoire stockant les instructions d'un programme informatique pour la mise en œuvre de ce procédé lorsque ces instructions sont exécutées par le processeur. La présente invention vise aussi un tel programme informatique (dont l'algorithme général peut être illustré par la figure 3 décrite ci-après).

### Brève description des dessins

D'ailleurs, d'autres avantages et caractéristiques de l'invention apparaitront à la lecture de la description d'exemples de réalisation présentés ci-après, et à l'examen des dessins annexés, dans lesquels :
**La** **figure 1**
   [Fig. 1] représente un individu portant un dispositif médical d'aide à la perception d'environnement objet de l'invention selon un premier exemple de mode de réalisation ;
**La** **figure 2**
   [Fig. 2] est une vue en perspective d'une partie du dispositif objet de l'invention en référence à la figure 1 ;
**La** **figure 3**
   [Fig. 3] correspond à un ordinogramme de l'algorithme général du procédé objet de l'invention en référence à la figure 1 ;
**La** **figure 4**
   [Fig. 4] illustre schématiquement un circuit de traitement notamment, du dispositif présenté sur la figure 1 ;
**La** **figure 5**
   [Fig. 5] représente un individu portant un dispositif médical d'aide à la perception d'environnement, selon un deuxième exemple de mode de réalisation ;
**La** **figure 6**
   [Fig. 6] représente un individu 5, porteur d'un dispositif au sens de l'invention, observant un environnement face à lui ;
**La** **figure 7**
   [Fig. 7] illustre schématiquement un des jeux d'actuateurs mécaniques ;

### Description détaillée de l'invention

La figure 1 représente un dispositif d'aide à la perception d'environnement typiquement dans l'espace frontal d'un utilisateur. Le dispositif est porté par un utilisateur 5 aveugle ou malvoyant, ce dispositif comportant un support mécanique 1 intégrant une caméra grand-angle 6 et un module de suivi oculaire 7. Plus précisément, dans le cas où les yeux de l'utilisateur sont tous les deux encore en mouvement et qu'un suivi oculaire est alors possible sur chaque œil, le dispositif peut comporter, comme illustré sur la figure 1, deux caméras grand-angle 6, chacune pour filmer l'environnement devant chaque œil de l'utilisateur, et deux modules de suivi oculaire 7, chacun pour suivre les mouvements d'un œil.

Typiquement, les deux caméras grand-angle, notamment, sont montées en positions respectives fixes sur le support 1, et les zones sélectionnées dans les images grand-angle se recouvrent partiellement pour assurer une stéréoscopie, permettant un ressenti 3D. A cet égard, le dispositif comporte en outre deux jeux d'actuateurs mécaniques 3 séparés spatialement comme illustré sur la figure 1.

Les caméras 6 et modules de suivi 7 sont reliés par liaison filaire à un circuit de traitement 2 pilotant les deux jeux d'actuateurs mécaniques 3.

Dans l'exemple représenté, le support mécanique 1 est placé sur la tête de l'utilisateur 5 tandis que les deux jeux d'actuateurs mécaniques 3 sont positionnés sur le torse de l'utilisateur 5. Les mouvements des yeux de l'utilisateur 5 sont mesurés par le biais des modules de suivi oculaire 7 en temps réel. Par ailleurs, on prévoit à titre d'exemple ici de sélectionner une zone dans les images acquises qui correspond à la direction courante du regard de l'utilisateur. Alternativement, on peut monter des caméras (pas nécessairement grand-angle) sur des axes respectifs pivotant et orienter les caméras en direction du regard courant de l'utilisateur. Alternativement encore, on peut prévoir une unique caméra grand-angle mais avec un suivi oculaire pour chaque œil, et deux zones sélectionnées dans l'environnement filmé, la stéréoscopie étant assurée encore par un recouvrement partiel de ces deux zones.

Dans la réalisation où l'environnement est filmé par une ou plusieurs caméras de type « grand-angle », la focale de telles caméras est choisie pour couvrir dans les images acquises ce à quoi correspondrait le champ visuel de l'utilisateur 5, s'il était valide.

Les mouvements des yeux de l'utilisateur 5 sont mesurés simultanément à la prise de vue de l'environnement par les caméras grand-angle 6. Plus précisément, comme illustré sur la figure 6, les mesures réalisées par les modules de suivi oculaire 7 sont analysées par le circuit de traitement 2 afin de sélectionner, en fonction de la mesure de la direction D du regard (pour chaque œil), la zone Z de pixels adéquate de l'image courante de l'environnement E qui vient d'être acquise par la caméra grand-angle 6.

Les mesures réalisées peuvent être envoyées au circuit de traitement 2 par le biais d'une liaison sans fil. En effet, une liaison sans fil dans cette application permet moins de gêne et une plus grande liberté de mouvements pour l'utilisateur 5, le circuit de traitement 2 et les actuateurs mécaniques 3 se trouvant sur le torse (ou le dos typiquement) de l'utilisateur 5.

Le circuit de traitement 2 traite les données reçues et envoi les instructions aux actuateurs mécaniques AM des jeux 3.

Les actuateurs mécaniques 3 positionnés sur le buste stimulent la peau de l'utilisateur 5 en fonction des instructions reçues pas le circuit de traitement 2. Ils « reproduisent » sur la peau les images modifiées après le traitement des données dans le circuit de traitement 2.

En pratique, le circuit de traitement 2 convertit une intensité de pixel (niveau de gris par exemple) de chaque pixel en intensité mécanique de stimulation par un actuateur. On peut associer en outre une fréquence de vibration particulière à chaque couleur de pixel (par exemple une fréquence basse pour le rouge et une fréquence plus haute pour le bleu). Par exemple aussi, on peut assigner une fréquence de vibration croissante aux différentes couleurs depuis le rouge jusqu'au violet, dans les couleurs du spectre visible.

La figure 2 représente une vue en perspective du support mécanique 1, avec une face 9 et deux branches 8 maintenues à la face 9, par exemple solidaires mécaniquement à cette dernière par une liaison charnière (non représentée). Deux modules de suivi oculaire 7 se situent vers les yeux typiquement pour filmer les pupilles et suivre leurs mouvements (souvent par reconnaissance de forme que met en œuvre un module informatique des images ainsi acquises) et, de là, déterminer une direction du regard de l'utilisateur. En revanche, les deux caméras grand-angle 7 sont positionnées en direction de l'environnement (selon les axes physiologiques des yeux du sujet).

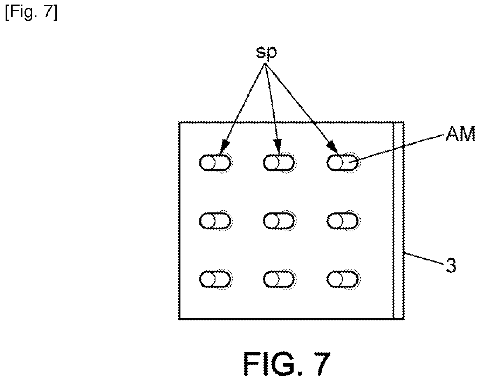

La figure 3 représente un ordinogramme résumant des étapes possibles d'un procédé mis en œuvre par le circuit de traitement 2. Un tel procédé peut comporter, à l'étape S1, l'acquisition d'une image courante par une caméra 6. A l'étape S2, un suivi oculaire (ou « eye tracking ») est mis en œuvre pour déterminer une direction courante du regard de l'utilisateur (à partir des mesures par suivi oculaire de la direction de regard de l'utilisateur 5 par le ou les module(s) de suivi oculaire 7). Une mise en cohérence du champ visuel avec l'orientation du regard est ainsi faite. A l'étape S3, en fonction de cette direction courante du regard, on sélectionne dans l'image acquise de l'environnement E (comme présenté ci-dessus en référence à la figure 6) une zone Z correspondant à la direction du regard D. Plus particulièrement, on sélectionne les signaux de pixels de cette zone Z pour les convertir à l'étape S4 en signaux de commande sp des actuateurs mécaniques AM (des jeux d'actuateurs 3 tels qu'illustrés sur la figure 7). Il peut s'agir d'éléments piézoélectriques ou de MEMS comme présenté plus haut, commandés par des signaux électriques sp en vue d'une mise en vibration de ces éléments :
- A une amplitude de vibration fonction de l'intensité de pixel (niveau de gris, par exemple), et
- A une fréquence de vibration fonction du type de couleur (à trois niveaux de fréquence pour bleu, vert ou rouge par exemple).

A l'étape S5, on envoie ensuite ces signaux de commande aux actuateurs mécaniques AM.

Bien entendu, ces étapes S1 à S5 sont réalisées pour chaque œil (et donc chaque image courante de caméra), et successivement pour toutes les images successivement acquises, en temps réel.

Les deux images vibratoires des deux actuateurs mécaniques 3 à la peau se veulent les plus proches possible de celles qu'auraient captées les rétines si le sujet avait été valide. Une telle réalisation permet de réapprendre l'espace environnant à l'individu 5.

La figure 4 représente le dispositif sous forme schématique comportant les caméras grand-angle 6 et les modules de suivi oculaire 7, ainsi que le circuit de traitement 2 comportant ici:
- une interface d'entrée IN pour recevoir les signaux des équipements 6 (pixels d'image courante) et 7 (mesure de direction du regard),
- une mémoire MEM pour stocker au moins temporairement des données correspondant à ces signaux, ainsi que des instructions d'un programme informatique au sens de l'invention, pour la mise en œuvre du procédé présenté ci-avant en référence à la figure 3,
- un processeur PROC capable de coopérer avec l'interface d'entrée IN et avec la mémoire MEM pour lire et exécuter les instructions du programme informatique et délivrer ainsi des signaux de commande des actuateurs AM, via
- une interface de sortie OUT reliée aux jeux d'actuateurs 3.

La figure 5 représente un dispositif d'aide à la perception spatiale d'environnement, porté par un utilisateur 5 sur la tête. Le dispositif comprend un support mécanique 1 s'étendant de part et d'autre de la tête et deux actuateurs mécaniques 3 positionnés sur le front de l'individu 5. Le support mécanique 1 comprend une face 9 et deux branches 8 maintenues à la face comme illustré la figure 2. Contrairement au dispositif illustré à la figure 1, le circuit de traitement 2 est intégré au support mécanique 1. Le support mécanique 1 prend la forme de casque permettant une liberté totale pour l'utilisateur 5. Ici, une liaison filaire courte entre le support mécanique 1 et les actuateurs mécaniques 3 peut être prévue dans ce mode de réalisation. En effet, elle ne limite pas l'utilisateur 5 dans ses mouvements et le dispositif est dans son intégralité sur la tête de l'utilisateur.

Bien entendu, la présente invention ne se limite pas aux exemples de réalisation décrits ci-avant ; elle s'étend à d'autres variantes.

Ainsi par exemple, on a décrit ci-avant une fréquence de vibration propre à une couleur. En variante, on peut choisir pour une image noir et blanc, une fréquence de vibration plus ou moins élevée en fonction du niveau de gris de chaque pixel.

Précédemment, on a défini une répartition des actuateurs du jeu d'actuateurs selon une forme bidimensionnelle correspondant à la forme de champ visuel que prend la zone d'image sélectionnée, avec en particulier un nombre d'actuateurs dans chaque jeu correspondant au nombre de pixels de la zone d'image sélectionnée. Néanmoins, en variante, il est possible de rendre actifs progressivement des actuateurs du jeu en nombre croissant (en partant du centre du jeu d'actuateurs jusqu'à la totalité des actuateurs du jeu), au fur et à mesure que l'utilisateur s'habitue au dispositif. En variante encore, il est possible de moyenner les amplitudes de vibration (ou autre stimuli) de n actuateurs parmi les N actuateurs du jeu, puis d'affiner ensuite la définition de perception en faisant décroitre n, au fur et à mesure encore que l'utilisateur s'habitue au dispositif. Bien entendu, pour favoriser rapidement cette adaptation, il est possible aussi de corriger (informatiquement) et atténuer ainsi des aberrations oculomotrices initiales, pour atténuer les saccades par exemple.

## Revendications

1. Dispositif d'aide à la perception d'environnement pour des utilisateurs aveugles ou malvoyants, le dispositif comportant :
- au moins un jeu d'actuateurs mécaniques destinés à être en contact avec la peau d'un utilisateur,
- au moins une caméra numérique agencée pour acquérir une image numérique courante d'un environnement face à l'utilisateur,
- un circuit de traitement connecté à la caméra pour recevoir des signaux de pixels de l'image numérique acquise et convertir une partie au moins des signaux de pixels en signaux de commande alimentant chacun un actuateur mécanique du jeu d'actuateurs,
**Caractérisé en ce que** le dispositif comporte en outre au moins un module de suivi oculaire d'au moins un œil de l'utilisateur pour identifier une direction de regard de l'utilisateur,
Et **en ce que** le circuit de traitement est agencé pour sélectionner dans l'environnement filmé par la caméra une zone d'image courante acquise qui est fonction de la direction de regard, et convertir les signaux de pixels de ladite zone en signaux de commande alimentant chacun un actuateur du jeu pour stimuler la peau de l'utilisateur.

2. Dispositif selon la revendication 1, **caractérisé en ce que** le circuit de traitement est agencé pour sélectionner dans une image courante acquise une zone limitée de l'image courante qui est fonction de la direction de regard, et convertir les signaux de pixels de ladite zone limitée en signaux de commande alimentant chacun un actuateur du jeu pour stimuler la peau de l'utilisateur.

3. Dispositif selon l'une des revendications 1 et 2, **caractérisé en ce qu'**il comporte :
- deux jeux d'actuateurs mécaniques destinés à être en contact avec la peau d'un utilisateur, et
- deux modules de suivi oculaire des yeux respectifs de l'utilisateur, pour définir deux zones dans l'image courante et convertir les signaux de pixels de chacune des deux zones en deux jeux respectifs de signaux de commande alimentant les deux jeux respectifs d'actuateurs.

4. Dispositif selon l'une des revendications précédentes, **caractérisé en ce que** la caméra et le module de suivi oculaire, au moins, sont montés sur un support mécanique commun, le module de suivi oculaire étant orienté vers un œil de l'utilisateur, la caméra étant orientée pour acquérir une image numérique courante d'un environnement face à l'utilisateur.

5. Dispositif selon l'une des revendications précédentes, **caractérisé en ce qu'**au moins une connexion entre le circuit de traitement et les actuateurs est une liaison sans fil pour la transmission des signaux de commande des actuateurs.

6. Dispositif selon l'une des revendications précédentes, **caractérisé en ce que** chaque actuateur est agencé pour stimuler la peau de l'utilisateur selon une intensité fonction du signal de commande reçu, et les signaux de commande sont fonctions chacun d'une intensité de couleur de pixel.

7. Dispositif selon la revendication 6, **caractérisé en ce que** chaque actuateur est mobile en vibration pour stimuler la peau de l'utilisateur par des vibrations cycliques, la fréquence des vibrations d'un actuateur étant fonction d'un type de couleur de pixel.

8. Dispositif selon l'une des revendications précédentes, **caractérisé en ce que** la zone d'image est de forme générale correspondant à une forme de champ visuel de l'œil suivi par le module de suivi oculaire, et **en ce que** les actuateurs du jeu d'actuateurs sont répartis selon une forme bidimensionnelle correspondant à ladite forme de champ visuel.

9. Procédé mis en œuvre par un circuit de traitement d'un dispositif selon l'une des revendications précédentes, **caractérisé en ce qu'**il comporte :
- recevoir des signaux de pixels de l'image numérique acquise,
- recevoir une mesure par suivi oculaire d'une direction de regard de l'utilisateur,
- déterminer une délimitation dans l'image acquise d'une zone correspondant à la direction du regard, et sélectionner les signaux de pixels de ladite zone,
- convertir les signaux de pixels de ladite zone en des signaux de commande destinés à alimenter chacun un actuateur du jeu d'actuateurs mécaniques, le nombre de pixels dans ladite zone correspondant à un nombre d'actuateurs que comporte le jeu d'actuateurs mécaniques,
- transmettre les signaux de commande respectifs aux actuateurs dudit jeu.

10. Programme informatique comportant des instructions pour la mise en œuvre du procédé selon la revendication 9, lorsque lesdites instructions sont exécutées par un circuit de traitement d'un dispositif d'aide à la perception d'environnement.

## Patentansprüche

1. Vorrichtung zur Verbesserung der Umgebungswahrnehmung für blinde oder sehbehinderte Benutzer, wobei die Vorrichtung aufweist:
- mindestens ein Set mechanischer Aktuatoren, die bestimmt sind, mit der Haut eines Benutzers im Kontakt zu sein,
- mindestens eine digitale Kamera, die eingerichtet ist, um ein laufendes digitales Bild von einer Umgebung gegenüber dem Benutzer zu erfassen,
- einen Verarbeitungskreis, der an die Kamera angeschlossen ist, um Pixelsignale des erfassten digitalen Bildes zu empfangen und mindestens einen Teil der Pixelsignale in Steuersignale umzuwandeln, die jeweils einen mechanischen Aktuator des Aktuatorsets versorgen,
**dadurch gekennzeichnet, dass** die Vorrichtung ferner mindestens ein Augenverfolgungsmodul mindestens eines Auges des Benutzers aufweist, um eine Blickrichtung des Benutzers zu identifizieren,
und dass der Verarbeitungskreis eingerichtet ist, um in der von der Kamera gefilmten Umgebung eine erfasste laufende Bildzone auszuwählen, die von der Blickrichtung abhängt, und die Pixelsignale der Zone in Steuersignale umzuwandeln, die jeweils einen Aktuator des Sets versorgen, um die Haut des Benutzers zu stimulieren.

2. Vorrichtung nach Anspruch 1, **dadurch gekennzeichnet, dass** der Verarbeitungskreis eingerichtet ist, um in einem erfassten laufenden Bild eine begrenzte Zone des laufenden Bildes auszuwählen, die von der Blickrichtung abhängt, und die Pixelsignale der begrenzten Zone in Steuersignale umzuwandeln, die jeweils einen Aktuator des Sets versorgen, um die Haut des Benutzers zu stimulieren.

3. Vorrichtung nach einem der Ansprüche 1 und 2, **dadurch gekennzeichnet, dass** sie aufweist:
- zwei Sets mechanischer Aktuatoren, die bestimmt sind, mit der Haut eines Benutzers im Kontakt zu sein, und
- zwei Augenverfolgungsmodule der jeweiligen Augen des Benutzers, um im laufenden Bild zwei Zonen zu definieren und die Pixelsignale von jeder der zwei Zonen in zwei jeweilige Steuersignalsets umzuwandeln, die die zwei jeweiligen Aktuatorsets versorgen.

4. Vorrichtung nach einem der vorangehenden Ansprüche, **dadurch gekennzeichnet, dass** mindestens die Kamera und das Augenverfolgungsmodul auf einem gemeinsamen mechanischen Träger angebracht sind, wobei das Augenverfolgungsmodul zu einem Auge des Benutzers ausgerichtet ist, wobei die Kamera ausgerichtet ist, um ein laufendes digitales Bild von einer Umgebung gegenüber dem Benutzer zu erfassen.

5. Vorrichtung nach einem der vorangehenden Ansprüche, **dadurch gekennzeichnet, dass** mindestens eine Verbindung zwischen dem Verarbeitungskreis und den Aktuatoren eine drahtlose Verbindung für die Übertragung der Steuersignale der Aktuatoren ist.

6. Vorrichtung nach einem der vorangehenden Ansprüche, **dadurch gekennzeichnet, dass** jeder Aktuator eingerichtet ist, um die Haut des Benutzers in einer Intensität in Abhängigkeit von dem empfangenen Steuersignal zu stimulieren und die Steuersignale jeweils von einer Pixelfarbintensität abhängen.

7. Vorrichtung nach Anspruch 6, **dadurch gekennzeichnet, dass** jeder Aktuator vibratorisch beweglich ist, um die Haut des Benutzers durch zyklische Vibrationen zu stimulieren, wobei die Frequenz der Vibrationen eines Aktuators von einem Pixelfarbtyp abhängt.

8. Vorrichtung nach einem der vorangehenden Ansprüche, **dadurch gekennzeichnet, dass** die Bildzone eine allgemeine Form hat, die einem Gesichtsfeld des Auges entspricht, das vom Augenverfolgungsmodul verfolgt wird, und dass die Aktuatoren des Aktuatorsets gemäß einer zweidimensionalen Form verteilt sind, die der Form des Gesichtsfelds entspricht.

9. Verfahren zur Umsetzung einer Vorrichtung nach einem der vorangehenden Ansprüche durch einen Verarbeitungskreis, **dadurch gekennzeichnet, dass** es aufweist:
- Empfangen der Pixelsignale des erfassten digitalen Bildes,
- Empfangen einer Messung einer Blickrichtung des Benutzers durch Augenverfolgung,
- Bestimmen einer Begrenzung einer Zone im erfassten Bild, die der Blickrichtung entspricht, und Auswählen der Pixelsignale der Zone,
- Umwandeln der Pixelsignale der Zone in Steuersignale, die bestimmt sind, jeweils einen Aktuator des Sets mechanischer Aktuatoren zu versorgen, wobei die Anzahl der Pixel in der Zone einer Anzahl von Aktuatoren entspricht, die das Set mechanischer Aktuatoren aufweist,
- Übertragen der jeweiligen Steuersignale an die Aktuatoren des Sets.

10. Softwareprogramm, das Befehle für die Durchführung des Verfahrens nach Anspruch 9 aufweist, wenn die Befehle von einem Verarbeitungskreis einer Vorrichtung zur Verbesserung der Umgebungswahrnehmung ausgeführt werden.

## Claims

1. A device for improving environmental perception for blind or visually-impaired users, the device comprising:
- at least one set of mechanical actuators intended to be in contact with the skin of a user,
- at least one digital camera arranged for acquiring a current digital image of an environment facing the user,
- a processing circuit connected to the camera for receiving signals from pixels of the acquired digital image and converting at least a part of the pixel signals into control signals each supplying a mechanical actuator of the set of actuators,
**characterized in that** the device furthermore comprises at least one eye-tracking module for at least one eye of the user for identifying a direction of gaze of the user,
and **in that** the processing circuit is arranged for selecting in the environment filmed by the camera an area of acquired current image which depends on the direction of gaze, and converting the signals from pixels of said area into control signals each supplying an actuator of the set for stimulating the skin of the user.

2. The device as claimed in claim 1, **characterized in that** the processing circuit is arranged for selecting within an acquired current image a limited area of the current image which depends on the direction of gaze, and converting the signals from pixels of said limited area into control signals each supplying an actuator of the set for stimulating the skin of the user.

3. The device as claimed in either of claims 1 and 2, **characterized in that** it comprises:
- two sets of mechanical actuators intended to be in contact with the skin of a user, and
- two eye-tracking modules for the respective eyes of the user, for defining two areas in the current image and converting the signals from pixels of each of the two areas into two respective sets of control signals supplying the two respective sets of actuators.

4. The device as claimed in one of the preceding claims, **characterized in that** the camera and the eye-tracking module, at least, are mounted onto a common mechanical support, the eye-tracking module being oriented toward one eye of the user, the camera being oriented so as to acquire a current digital image of an environment facing the user.

5. The device as claimed in one of the preceding claims, **characterized in that** at least one connection between the processing circuit and the actuators is a wireless link for the transmission of the control signals for the actuators.

6. The device as claimed in one of the preceding claims, **characterized in that** each actuator is arranged for stimulating the skin of the user according to an intensity that is a function of the control signal received, and the control signals are each functions of an intensity of color of pixel.

7. The device as claimed in claim 6, **characterized in that** each actuator is mobile in vibration for stimulating the skin of the user by cyclical vibrations, the frequency of the vibrations of an actuator being determined by a type of color of pixel.

8. The device as claimed in one of the preceding claims, **characterized in that** the image area is of general shape corresponding to a shape of visual field of the eye tracked by the eye-tracking module, and **in that** the actuators of the set of actuators are distributed according to a two-dimensional shape corresponding to said shape of visual field.

9. A method implemented by a processing circuit of a device as claimed in one of the preceding claims, **characterized in that** it comprises:
- receive signals from pixels of the acquired digital image,
- receive a measurement by eye-tracking of a direction of gaze of the user,
- determine a bounding in the acquired image of an area corresponding to the direction of the gaze, and select the signals from pixels of said area,
- convert the signals from pixels of said area into control signals each intended to supply one actuator of the set of mechanical actuators, the number of pixels in said area corresponding to a number of actuators that the set of mechanical actuators comprises,
- transmit the respective control signals to the actuators of said set.

10. A computer program comprising instructions for the implementation of the method as claimed in claim 9, when said instructions are executed by a processing circuit of a device for improving environmental perception
